# FASCICULE DE BREVET EUROPEEN

(11) **EP 1 427 717 B1**
(45) Date de publication et mention de la délivrance du brevet: **22.11.2006**
(21) Numéro de dépôt: 02779635.8
(22) Date de dépôt: 16.09.2002
(51) Int. Cl.: C07D 311/72, A61K 31/355, C07D 407/14

(54) **BIOPRECURSEURS DESTINES A UNE APPLICATION PERCUTANEE**
BIOLOGISCHE VORSTUFEN FÜR DIE PERKUTANE VERABREICHUNG
BIOPRECURSORS FOR PERCUTANEOUS APPLICATION

(30) Priorité: 17.09.2001 FR 0111982
(43) Date de publication de la demande: 16.06.2004
(73) Titulaire: Pierre Fabre Dermo-Cosmétique, 92100 Boulogne-Billancourt (FR); Université Paul Sabatier (Toulouse III), 31062 Toulouse Cedex 9 (FR)
(72) Inventeur: REDOULES, Daniel, F-31300 Toulouse (FR); BORDAT, Pascal, F-31320 Mervilla (FR); PERIE, Jean-Jacques, F-31320 Castanet (FR)
(74) Mandataire: Ahner, Francis
(86) Numéro de dépôt international: PCT/FR2002/003148
(87) Numéro de publication internationale: WO 2003/024952

(56) Documents cités:
- WO-A-00/58325
- WO-A-00/61189
- FR-A- 2 722 094
- CHEMICAL ABSTRACTS, vol. 109, no. 30, 1988 Columbus, Ohio, US; abstract no. 73692f, page 719; XP002201606 & JP 06 287470 A (ARAKAWA) 11 octobre 1994 (1994-10-11)
- C.DUVAL: "SCAVENGER EFFECT OF VITAMIN E" JOURNAL OF PHARMACEUTICAL SCIENCES, vol. 84, no. 1, janvier 1995 (1995-01), pages 107-110, XP002201605 USA

## Description

La présente invention se rapporte à une composition cosmétique ou pharmaceutique pour application cutanée, contenant un composé apte à libérer deux molécules actives (vitamines en particulier) par action d'une activité estérasique contenue dans la peau, l'espaceur pouvant introduire un effet additionnel (effet hydratant d'hydroxy acide par exemple).

Il a été précédemment montré, à partir de deux sources d'enzymes cutanées que cette enzyme était bien capable de reconnaître et d'hydrolyser des esters succiniques permettant ainsi un relargage lent de la substance active, sans effet d'accumulation (M.P. Mora et coll., *Chem Phys Lipids* (1999), **101,** 255-265, J. R. Trevithick et coll., *Biochem. Mol. Biol. Int.* (1999), **47,** 509-511).

La stratégie des bioprécurseurs a été précédemment utilisée pour la libération d'actifs, notamment dans les cas qui suivent:
- libération de rétinol à partir de son ester avec l'acide palmitique.

De nombreux composés sont utilisés en dermatologie, parmi lesquels on retiendra:
- les agents hydratants ou plus précisément les agents contrôlant la perspiration cutanée tels que les saccharides (glucose, sorbitol, acide hyaluronique), mais aussi le glycérol et les acides α-hydroxylés, ou même les céramides d'origine végétale, hydratantes par leur partie polaire. Parmi les hydroxy-acides, l'acide L-ascorbique occupe une place de choix, puisqu'il associe à cette première propriété d'autres effets: un effet antioxydant et aussi la capacité à stimuler la synthèse du collagène (et donc des fibres élastiques) par une augmentation spécifique du taux d'ARNm codant pour les trois chaînes pro-α. De plus, l'acide ascorbique active l'enzyme assurant la formation du collagène à partir du procollagène (S.R. Pinel et coll., *Arch. Dermatol*. (1987), **123**, 1684-1687);
- les agents antioxydants: on sait qu'ils sont essentiels pour éviter la dégradation des lipides sous l'action des radicaux ainsi que les autres dommages pour les biomolécules de la surface cutanée, dommages conduisant à la formation des rides. Parmi les principaux pièges radicalaires, on trouve les tocophérols, les flavonoïdes, l'acide ascorbique ainsi que les agents de chélation des métaux, qui bloquent les réactions d'oxydation catalysés par lesdits métaux;
- les agents de contrôle de la différenciation: le plus important est la vitamine A ou rétinol puisqu'une carence se traduit par une hyperkératinisation ainsi qu'une atrophie des glandes sébacées et sudorales. Le rétinol n'est pas utilisé comme tel mais après deux oxydations enzymatiques, la première réversible, le transformant en rétinal, la seconde irréversible le transformant en acide rétinoïque qui est la forme active agissant sur les récepteurs nucléaires. Le stockage est assuré sous forme d'esters linoléique et palmitique. Un apport peut être assuré sous forme d'esters ou sous forme de rétinol lié à un vecteur. On sait que l'action de l'acide rétinoique est de plusieurs ordres: activateur du métabolisme cellulaire et contrôle de la kératinisation, antioxydant et donc prévention de la formation des rides, action au niveau du derme sur le métabolisme des fibroblastes par deux effets: inhibition des enzymes dégradant le collagène (collagénases) et stimulation des glucosaminoglycanes qui augmentent la teneur en collagène et donc augmentation de l'élasticité de la peau;
- la vitamine D qui, outre son action sur le métabolisme phosphocalcique, exerce par l'intermédiaire de sa forme biologiquement active, le calcitriol, des effets sur la prolifération et la différenciation cellulaires. L'incubation de kératinocytes humains avec le calcitriol entraîne, en effet, une diminution de leur prolifération et l'induction de leur différenciation terminale (M F Holick et coll. *Arch. Dermatol*. (1987), **123**, 1677-1683). Le calcitriol est encore un immunosuppresseur puissant qui inhibe l'activation lymphocytaire et la production d'immunoglobulines (M Bagot et coll., *Br. J. Dermatol*. (1994), **130**, 424-431).

Dans l'art antérieur, des bioprécurseurs de dérivés de rétinoïde, en particulier sous forme d'esters de rétinol sont divulgué dans le document WO-00/53325. Le document FR 2 722 094 décrit des compositions destinées à activer le bronzage, associant notamment un caroténoïde et un tocophérol dans un même capsule la demande WO-00/61189 divulgue une composition hydrosoluble comprenant un diester porteur d'une partie terminale hydrophobe et d'une partie terminale hydrophile. **Enfin, la demande GB 2 285 805 divulgue des diesters symétriques d'acide carboxique et de stérols ou de vitamine, pour leur propriété antitumorale.**

L'utilisation directe de ces dérivés par voie topique se heurte à un certain nombre de difficultés, du fait de leur manque de stabilité dans le temps et à la lumière, et d'effets secondaires (pro-oxydants et irritation) résultant souvent de sur-concentrations locales des molécules actives.

D'où l'intérêt d'améliorer la biodisponibilité d'actifs véhiculés sous forme d'un précurseur les associant et évitant ainsi leurs effets néfastes dus à l'accumulation.

Or, les inventeurs; après une recherche d'expression par les kératinocytes, d'une activité lipasique ou cholestérol estérasique, ont identifié la lipase acide lysosomiale (*Human Lysosomial Acid Lipase* ou HLAL, Anderson R.A. *et al*., *J. Biol. Chem.,* **266**, 22479-84) et ont montré de manière surprenante que cette estérase était capable de couper certains précurseurs qui se présentaient sous forme d'actifs estérifiés.

En conséquence, la présente invention concerne un bioprécurseur de formule (I) 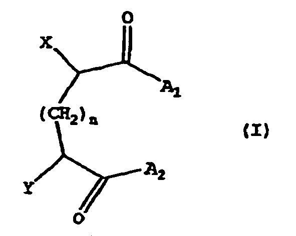 dans laquelle
A₁ et A₂ représentent chacun indépendamment l'un de l'autre un radical provenant d'une molécule susceptible d'être utilisée en dermatologie ou en cosmétologie et choisie parmi les céramides d'origine végétale, les tocophénols, les flavonoïdes, le rétinol, le rétinal, l'acide rétinoïque, X et Y représentent indépendamment l'un de l'autre un atome d'hydrogène, un groupe hydroxy ou un groupe (C₁-C₂₀) alkyle; et
n représente un nombre entier compris entre 0 et 10.

Dans un mode particulier de réalisation de l'invention A₁ et A₂ représentent chacun indépendamment l'un de l'autre un radical cholécalciféryle, rétinyle, tocophéryle.

Dans un mode particulièrement avantageux de réalisation de l'invention, A₁ représente un radical tocophéryle et A₂ représente un radical choisi dans le groupe comprenant les radicaux rétinyle, cholécalciféryle.

Dans un mode de réalisation encore plus avantageux selon l'invention, le composé de formule (I) est choisi dans le groupe constitué par le tocophéryl-rétinyl-succinate, et le tocophéryl-cholécaldiféryl-succinate.

La présente invention s'étend également à des compositions pharmaceutiques ou cosmétiques à usage topique contenant au moins un bioprécurseur de formule (I) associé à un véhicule approprié pour l'administration percutanée.

Conformément à la présente invention, lorsque ladite composition est appliquée sur la peau, le complexe subit une hydrolyse enzymatique de type estérase conduisant à la libération du principe actif, ledit principe actif étant libéré de façon retardée, sans effet d'accumulation dans les différentes couches de la peau.

Les compositions selon l'invention contiennent de 0,001 à 10% en poids, de préférence 0,01 % à 0,1% en poids, de bioprécurseurs par rapport au poids total de la composition.

Elles peuvent se présenter sous forme d'émulsion huile dans eau (H/E) ou eau dans huile (E/H). Elles peuvent encore se présenter sous forme de sphérules comme les liposomes, les nanocapsules ou les nanosphères.

Lorsque les compositions sont des émulsions, la proportion de la phase grasse va de 5 à 80 % en poids, de préférence de 5 à 50% en poids, par rapport au poids total de la composition. Les huiles, les émulsionnants et les coémulsionnants utilisés dans ces compositions, sous forme d'émulsion, sont choisis parmi ceux classiquement utilisés en cosmétique. L'émulsionnant et le co-émulsionnant sont présents, dans les compositions, en une proportion allant de 0,3 à 10% en poids, par rapport au poids total de la composition.

Les compositions selon l'invention peuvent également contenir des additifs cosmétiques ou dermatologiques acceptables. Ces additifs peuvent être, en particulier, des antioxydants, d'autres bioprécurseurs de ces antioxydants comme le δ-tocophérylglucopyranoside, des tensioactifs, des corps gras, des agents hydratants, des conservateurs, des parfums, des gélifiants, des chélateurs, des pigments comme l'oxyde de titane, des filtres et des vitamines libres.

Les bioprécurseurs de formule (I) sont préparés par des techniques connues de l'homme du métier.

Dans un mode de réalisation particulièrement avantageux du procédé, on fait réagir un composé de formule (II)

A₁ - H (II)

où A₁ est tel que défini précédemment,
avec un composé de formule (III) 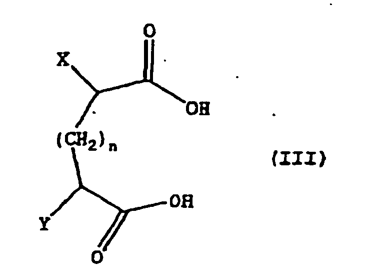 où X et Y sont tels que définis précédemment,
dans un mélange contenant un solvant comme le dichlorométhane anhydre, la triéthylamine, II, III le dicyclohexylcarbodiimide (DCC) et la diméthylaminopyridine (DMAP), on obtient un composé de formule (IV) 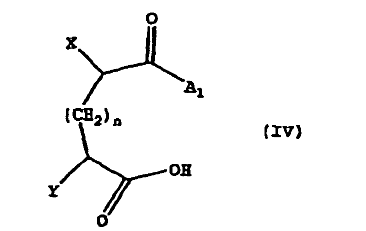 composé que l'on fait réagir dans les mêmes conditions que précédemment avec un composé de formule (V)

A₂ - H (V)

et on obtient le composé de formule (I).

Dans un autre mode de réalisation avantageux du procédé selon l'invention, on fait réagir le composé de formule (II) avec une forme activée du composé de formule (III), par exemple l'anhydride succinique.

Les avantages de cette stratégie des bioprécurseurs sont les suivants:
- stabilisation de l'actif grâce à la neutralisation de la fonction hydroxyle la plus réactive (vitamines D, E ou A). Du fait de l'importance de l'activité estérase dans les couches vivantes de l'épiderme et de sa quasi absence dans la couche cornée (U.K. Jain et coll., *Proceed. Intern. Symp. Control. Rel. Bioact. Mater*. (1995), 22, 702-703), les précurseurs qui représentent la forme stabilisée des principes actifs sont préservés tout au long de la lente migration au travers de la couche cornée laquelle correspond à la partie la plus exposée au stress oxydatif (UV et ozone) (G. Valacchi et coll., *FEBS Letters* (2000), **446**, 165-168);
- libération avec une vitesse contrôlée (prise en compte de la régulation très précise de la physiologie cutanée) et effet réservoir.

De plus, on obtient une libération ciblée à partir du stratum granulosum au niveau des membranes plasmiques des kératinocytes *(M. P. Mora et coll. , Chem. Phys. Lipids* (1999), **101**, 255-265), c'est-à-dire dès que débute la partie vivante de l'épiderme.

Le précurseur, après migration dans les premières couches de l'épiderme vivant c'est à dire au niveau du stratum granulosum, est reconnu en tant que pseudo-substrat par l'activité estérase impliquée, responsable de l'hydrolyse des deux fonctions ester. Deux effets conjugués, additifs voire synergiques, sont ainsi obtenus à partir d'une formulation unique, cette dernière facilitant la migration.

Ainsi, l'association tocophérol-acide ascorbique est particulièrement intéressante, puisque ce dernier régénère le tocophérol après son oxydation, augmentant ainsi son efficacité d'antioxydant.

La structure des précurseurs selon l'invention assure une bonne stabilité tout au long de la traversée de la couche et une libération au niveau des couches vivantes de l'épiderme des actifs avec une cinétique assurant une coupure effective et un effet avec rémanence dans le temps.

La pénétration à travers la peau est liée au Kd des composés (Agache P. *et coll., Ed. Tech. Encycl. Méd. Chir*. (1995), 12-235-C-30, 1-10); or, la structure des précurseurs selon l'invention permet également de moduler la pénétration des actifs pseudosubstrats grâce à la présence de X et Y qui représentent soit des groupes hydroxy donc hydrophiles, soit des groupes (C₁-C₂₀)alkyle donc lipophiles qui permettent d'obtenir un composé amphiphile susceptible de pénétrer en quantité appréciable par diffusion passive.

Les exemples qui suivent illustrent l'invention sans toutefois la limiter.

### Exemple 1: O-(4-oxo-4-{[2,5,7,8-tetramethyl-2-(4,8,12-trimethyltridecyl)-3,4-dihydro-2H-chromen-6-yl]oxy}butanoyl)retinol on tocophéryl-rétinyl-succinate (CV-105).

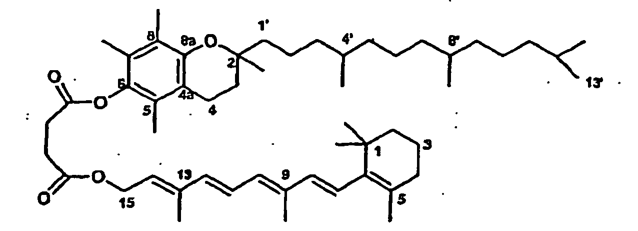

### 1.1. Méthyl 2,5,7,8-tetramethyl-2-(4,8,12-trimethyltridecyl)-3,4-dihydro-2H-chromen-6-yl succinate (CV-104).

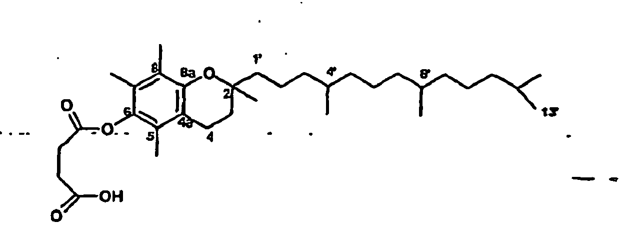

Deux méthodes de synthèse ont été développées soit à partir de l'anhydride succinique, soit de l'acide succinique.

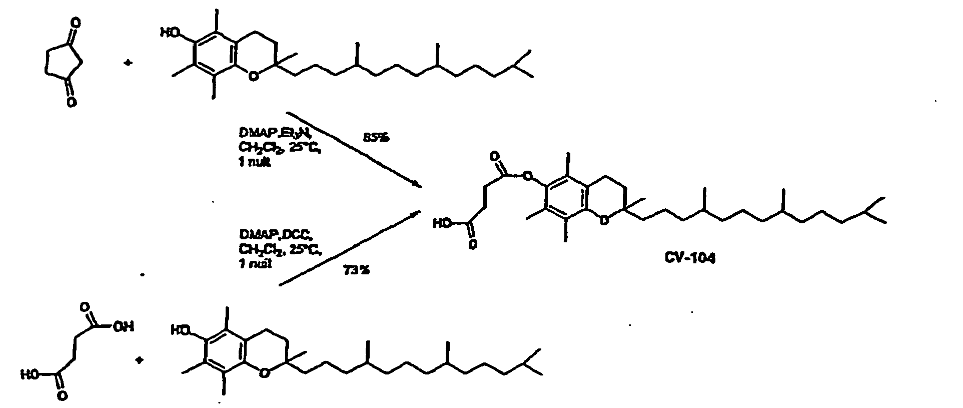

Selon la première méthode, l'α-tocophérol ou vitamine E (2,15 g, 5 mmol), l'anhydride succinique (750 mg, 7,5 mmol, 1,5 eq) sont dissous dans 20 ml de dichlorométhane (CH₂Cl₂) anhydre. La diméthylaminopyridine (DMAP) (305 mg, 2,5 mmol, 0,5 eq) et la triéthylamine anhydre (0,7 ml, 1 eq) y sont rajoutés et la réaction est suivie par chromatographie sur couche mince (CCM) (Et₂O/ether de pétrole (EP) 2:3 ou acétate d'éthyle/EP 1:1). La réaction est en général finie au bout d'une nuit de réaction, le mélange est donc filtré puis lavé avec de l'acide chlorhydrique (HCl) 5% aqueux. La phase organique est séchée sur MgSO₄ puis évaporée pour donner une huile jaune (3 g environ) à purifier.

Selon la deuxième méthode, l'acide succinique (590 mg, 5 mmol), le dicyclohexylcarbodiimide (DCC) (1,03 g, 1 eq) et le DMAP (61 mg, 0,5 mmol, 10%) sont dissous dans 20 ml de CH₂Cl₂ anhydre aux ultrasons pendant 10-15 minutes puis l'α-tocophérol ou vitamine E (2,15 g, 5 mmol, 1 eq) en solution dans 10 ml de CH₂Cl₂ anhydre y est additionné. La réaction est suivie par CCM (Et₂O/EP 2:3 ou AcOEt/EP 1:1). Le mélange est donc filtré puis lavé avec HCl 5% aqueux, la phase organique est séchée sur MgSO₄ puis évaporée pour donner une huile jaune à purifier.

Dans les deux cas le produit est purifié par flash-chromatographie (colonne 2,5 cm x 16 cm, éluant Et₂O/EP 2:3, fractions de 10-15 ml). Le dépôt de l'huile pâteuse peut être fait en dépôt solide ou dans CH₂Cl₂. La première méthode conduit à 2,26 g de CV-104 pur, soit 85% de rendement et la seconde à 1,95 g, soit 73% de rendement d'une huile jaune pâle qui durcit à -20°C et peut donner une poudre blanche.
Rf *(Et*_{*2*}*O*/*EP 2:3)*: 0,39.
RMN ¹H *(CDCl*_{*3*}*, 250 MHz)*: 2,87 (dt, 4H, H-(C_{ac succ}), ²J=21, ³J=6,6) ; 2,58 (t, 4H, H-(C₄, C₃), ³J=6) ; 2,08, 2,01, 1,97 (3s, 9H, CH₃-(C₅), CH₃-(C₇), CH₃-(C₈)), 1,6-1 (gros multiplet, 24H, CH₃-(C₁) + 9 x CH₂, 3 x CH tocophérol), 0,88, 0,84 (2s, 12H, 4 x CH₃ tocophérol) .
RMN ¹³C *(CDCl*_{*3*}*, 50 MHz)* : 177,51, 170,96 (s, C=O acide + C=O ₑₛₜₑᵣ) ; 149,48 (s, C₈ₐ) ; 140,44 (s, C₆) ; 126,73 (s, C₇) ; 125,00 (s, C₅) ; 123,10 (s, C₈) ; 117,45 (s, C₄ₐ) ; 75,11 (s, C₂) ; 39,42 (t, C_{1'}) ; 37,45 (t, C_{7'} + C_{9'} + C_{3'} + C_{5'} + C_{11'} ) ; 32,83 (d, C_{8'} + C_{4'} + C_{12'}) ; 28,97, 28,68, 24,88, 24,50, 21,08, 20,64 (C_{10'} + C_{6'} + C_{2'} + C₄ + CH₂ ac succ) ; 28,04 (CH₃-(C₂)) ; 22,69 (C_{13'} ) ; 19,74, 12,7, 12,18, 12,10, 11,99 (CH-(C_{12'} + C_{8'} + C_{4'}) + CH₃-(C₈ + C₇ + C₅) + CH₃-(C₁)).
IR (*NaCl*): 2924, 2856, 2735, 2638, 2540, 1746, 1694, 1455, 1421, 1378, 1323, 1246, 1155, 1106, 919, 853 , 799, 728.
MS (CI, NH₃): 548 ([MNH₄]⁺, 100) ; 531 ([MH]⁺, 11.3) ; 530 ([M]⁺, 3, 4) .
UV (CH₃CN): 286 (0,047 à 14 µg/ml) ; 203 (0,61 à 14 µ g/ml).
EA pour C₃₃H₅₄O₅ (530, 78) : calc. C 74,67, H 10,25; exp. C 74, 85, H 10,26.

### 1.2. Tocophéryl-rétinyl-succinate (CV-105)

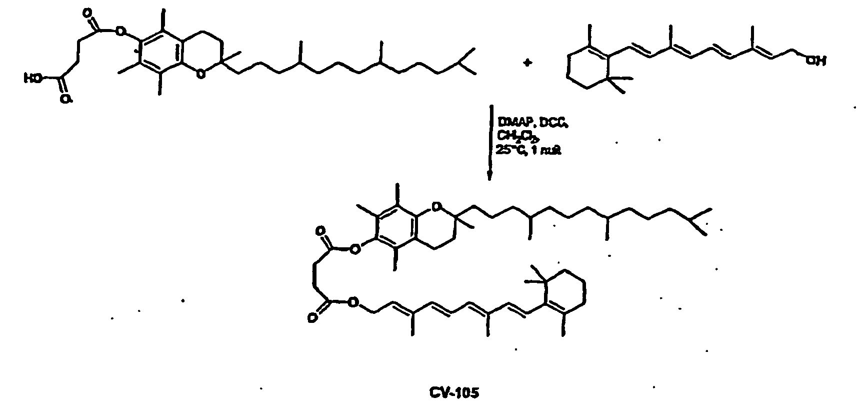

CV-104 préparé à l'exemple 1.1 (356 mg, 0, 67 mmol) est dissous dans 20 ml de CH₂Cl₂ anhydre, le DCC (138 mg, 1 eq), le DMAP (20 mg) y sont rajoutés directement. Après 10 minutes il se forme déjà un précipité de dichlorohexylurée (DCU), l'anhydride de CV-104 ayant dû se former. Après 20 minutes le rétinol-vitamine A (192 mg, 1 eq) en solution dans 5 ml de CH₂Cl₂ anhydre est rajouté. La réaction conduite à l'abri de la lumière est suivie en CCM (CH₂Cl₂ /EP 2:3), un nouveau produit moins polaire se forme. Après une nuit de réaction, le solvant est filtré puis évaporé. Le produit est purifié sur flash chromatographie avec AcOEt/EP (2:3) et on obtient une huile jaune (266 mg-50% rendement). Une deuxième purification est faite en HPLC (20 g silice 35, éluant Et20/ EP 1:9).
A partir de 130 mg du premier lot purifié, 72 mg d'une huile translucide (CV-105) sont obtenus, soit 28% de rendement.

### Exemple 2: Tocophéryl-colecalciferyl-succinate (CV-125)

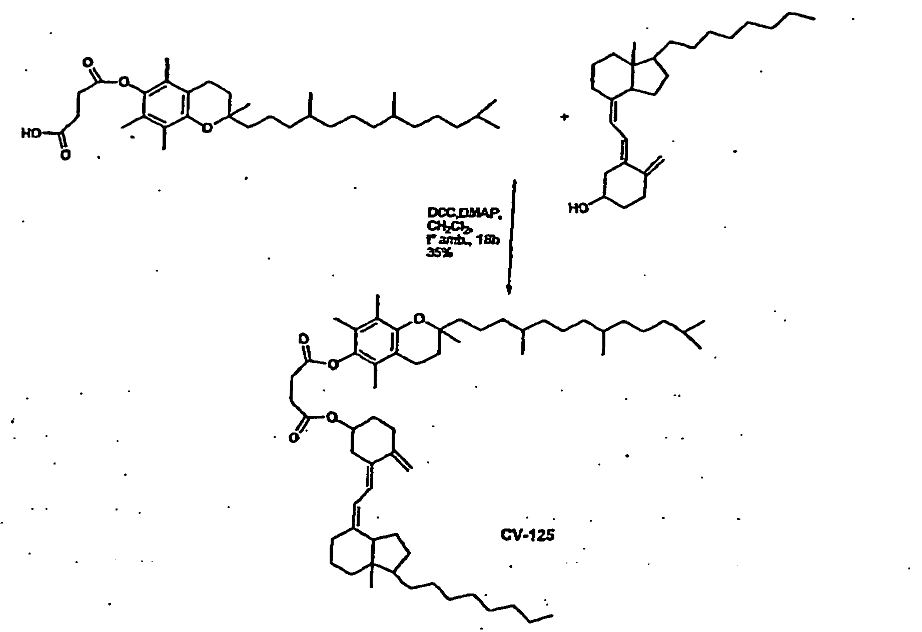

CV-104 préparé à l'exemple 1.1 (423 mg, 0,798 mmol) est dissous dans 10 ml de CH₂Cl₂-anhydre, le DCC (181 mg, 1,1 eq), le DMAP (20 mg) y sont rajoutés directement. Après 20 minutes la vitamine D₃ (307 mg, 1 eq) en solution dans 10 ml de CH₂Cl₂ anhydre est rajoutée. La réaction conduite à l'abri de la lumière est suivie en CCM (AcOEt /EP 2:8 ou 5:95), un nouveau produit moins polaire se forme. Après une nuit de réaction, le solvant est filtré puis évaporé. Le produit purifié sur flash chromatographie avec AcOEt/EP (5:95) comme éluant, colonne 2 x 12 cm, fraction de 6-8 ml. Les fractions 4 à 10 sont évaporées pour conduire à 253 mg d'une huile translucide de tocophéryl-calciféryl-succinate (CV-125) (35% de rendement).
Rf (AcOEt/EP 2:8): 0,91; (AcOEt/EP 5:95): 0, 32.
RMN ¹H *(CDCl*_{*3*}*, 250 MHz)*: 6,22 (d, 1H, H-(C₇), ³J₇₋₈=11,2); 6,04 (d, 1H, H-(C₈), ³J₈₋₇=11,2); 5,07 (s, 1H, CH₂-(C₄)); 4,98 (m, 1H, H-(C₁)); 4,85 (d, 1H, CH₂-(C₄)); 2,92 (dd, 2H, CH_{2-succ}, ²J=16 , ³J=6,8); 2,75 (m, 3H, CH_{2-succ} + CH-(C₄), ²J=16 , ³J=6,8); 2,60 (m, 2H, CH-(C₆)+CH-(C₄)); 2,4 (m, 2H, CH-(C₆)+H-(C₁₇)); 2,09, 2,03, 1,99 (3s, 9H, CH₃₋(C₅), CH₃-(C₇), CH₃-(C₈)), 1.,3-0,9 (gros multiplet, 47H, 11 x CH_{2-vitE}, 3 x CH-(C_{12'}, C_{4'}, C_{8'})+11x CH₂-ᵥᵢₜₙ₃); 0,89 et 0,87 (2s, 21H, 4 x CH₃ -(C_{12'}, C_{4'}, C_{8'})+C₂₅); 0,55 (s, 3H, CH₃-(C₁₃)).
RMN ¹³C *(CDCl*_{*3*}*, 50 MHz)*: 171,72, 171,07 (s, 2 x C=O ₑₛₜₑᵣ); 149,47 (s, C₅); 144,64 (s, C₆); 142,55 (s, C₈ₐ); 140,50 (s, C₉); 134,23 (s, C₄); 126,76 (s, C₇); 125,00 (s, C₅); 123,06 (s, C₈); 122,61 (d, C₇); 117,53 (d, C₈); 117,38 (s, C₄ₐ); 112,81 (t, CH₂-(C₄)); 75,08 (s, C₂); 72,28 (d, C₁); 56,66 (d, C₁₇); 56,43 (d, C₁₄); 45,97 (s, C₁₃); 42,19 (t, C₁₂); 36,20, 32, 84, 32, 77 (d, C_{8'}+C_{4'}+C_{12'}); 40,62, 39,57, 39,44, 37,47, 32,23, 31,99, 31,12, 29,50, 29,12, 28,96, 28,08, 27,76 (t, C₁ '+C₃+C_{7'}+C_{9'}+C_{3'}+C_{5'}+C_{11'}+C₁₅+C₁₀+C₆ +C₂+C₃+C₁₈₋₂₃+2 x CH₂ ac succ), 24,89, 24,52, 23,95, 23,64, 22,29, 21,11, 20, 67 (t, C_{10'}+C_{6'}+C_{2'}+C₄+C₂₄+C₁₆+C₁₁) ; 22,91, 22,81, 22, 71 (q, CH₃-(C₂+2 x C_{13'}); 19,76, 18,92, 13,05, 12,19, 12,05, 11,89 (q, CH₃-(C_{8'}+C_{4'})+CH₃-(C₈ +C₇+C₅)+C₂₅+ CH₃-(C₂)).
IR (NaCl): 2950, 2867, 2119, 1758, 1736, 1463, 1412, 1377, 1240, 1202, 1146, 1110, 1079, 996, 734.
MS *(CI, NH*_{*3*}) : 914 ([MNH₄]⁺, 100); 897 ([MH]⁺, 2,86) ; 896 ([M]⁺, 2.71).
EA pour C₆₀H₉₆O₅ (896): calc. C 80,30, H 10,78; exp. C 77,36, H 10,26.

### Exemple 3: Hydrolyse enzymatique

### 3.1. Mode opératoire

Sur une boite contenant une lignée de kératinocytes HaCaT (75 cm²) dans 15 ml de sérum seul on dépose 150 µl de solution 1 mM dans le diméthylsulfoxyde (DMSO). Les cellules sont placées dans l'incubateur à 37°C pendant 24 heures. On procède à l'extraction du milieu par 15 ml d'acétate d'éthyle. La phase organique est ensuite isolée et évaporée.

Les cellules sont extraites par sonication dans 2 x 15 ml d'un mélange glacé constitué de chloroforme et de méthanol (1:2,5). Après centrifugation la phase organique prélevée est évaporée à sec. Pour chaque essai un contrôle est réalisé sur une boite contenant des cellules et un milieu sans substrat pour tenir compte d'une éventuelle dégradation chimique du substrat.

La présence d'une activité estérase dans les kératinocytes humains est vérifiée grâce à un substrat, le 4-méthyl-unbelliféryl-palmitate. Le précurseur selon l'invention, utilisé comme pseudo-substrat, est le tocophéryl-rétinyl-succinate préparé selon l'exemple 1.

### 3.2. Résultats

Ils sont rassemblés dans le tableau qui suit:

| Substrat | Taux d'hydrolyse en 24 heures d'incubation | Partie libérée |
|---|---|---|
| 4-méthyl-umbelliféryl-palmitate | 2,7 ± 2% | 4-méthyl-umbelliférone |
| Tocophéryl-rétinyl-succinate | 5 % ± 3% | vitamines A et E |

Les valeurs obtenues montrent une libération simultanée des vitamines E et A à partir du tocophéryl-rétinyl-succinate. Ces résultats confirment une très bonne cinétique de coupure dans les cas d'esters succiniques par les estérases kératinocytaires. De plus, on constate une libération avec effet réservoir puisqu'on retrouve dans la prise d'essai, le précurseur non dégradé.

## Revendications

1. Bioprécurseur de formule (I) 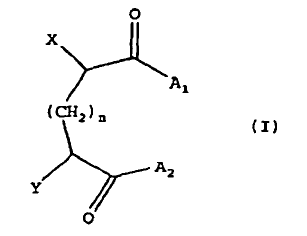 dans laquelle
A₁ représente un radical tocophéryle,
A₂ représente un radical provenant d'une molécule susceptible d'être utilisée en dermatologie ou en cosmétologie et choisie parmi les céramides d'origine végétale, les flavonoïdes, le rétinol, le rétinal, l'acide rétinoïque, la vitamine D, le calcitriol,
X et Y représentent indépendamment l'un de l'autre un atome d'hydrogène, un groupe hydroxy ou un groupe (C₁-C₂₀)alkyle; et
n représente un nombre entier compris entre 0 et 10.

2. Bioprécurseur selon la revendication 1, **caractérisé en ce que** A₁ représente un radical tocophéryle et A₂ représente un radical choisi dans le groupe comprenant les radicaux rétinyle, cholécalciféryle.

3. Bioprécurseur selon l'une quelconque des revendications 1 à 2, **caractérisé en ce qu'**il est choisi dans le groupe constitué par le tocophéryl-rétinyl-succinate et le tocophéryl-cholecalciféryl-succinate.

4. Bioprécurseur selon l'une quelconque des revendications 1 à 3, **caractérisé en ce qu'**il est choisi parmi

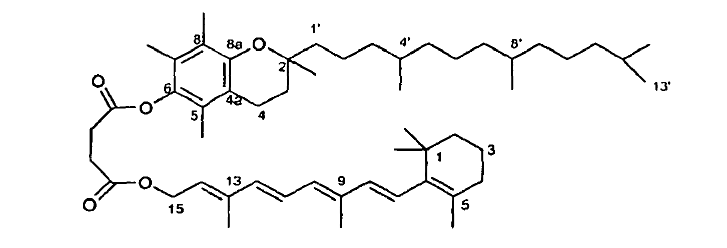

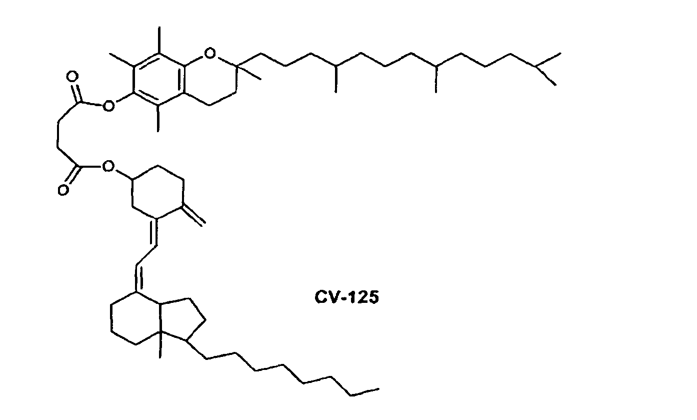

5. Compositions pharmaceutiques **caractérisées en ce qu'**elles contiennent au moins un bioprécurseur selon l'une quelconque des revendications 1 à 4, associé à un véhicule approprié pour l'administration percutanée.

6. Compositions pharmaceutiques selon la revendication 5, **caractérisées en ce qu'**elles contiennent de 0,001 à 10% en poids, de préférence de 0,01 à 0,1% en poids, de bioprécurseurs par rapport au poids total de la composition.

7. Compositions pharmaceutiques selon l'une quelconque des revendications 5 et 6, **caractérisées en ce qu'**elles se présentent sous forme d'émulsion huile dans eau (H/E) ou eau dans huile (E/H).

8. Compositions pharmaceutiques selon l'une quelconque des revendications 5 et 6, **caractérisées en ce qu'**elles se présentent sous forme de sphérules.

9. Compositions pharmaceutiques selon la revendication 7, **caractérisées en ce que** la proportion de la phase grasse va de 5 à 80% en poids, de préférence de 5 à 50% en poids, par rapport au poids total de la composition.

10. Compositions pharmaceutiques selon l'une quelconque des revendications 6 à 9, **caractérisées en ce qu'**elles contiennent en outre des additifs cosmétiques ou dermatologiques acceptables.

11. Procédé de préparation des bioprécurseurs selon l'une quelconque des revendications 1 à 3, **caractérisé en ce que** l'on fait réagir un composé de formule (II)
A₁ - H (II)
où A₁ est tel que défini dans la revendication 1,
avec un composé de formule (III) 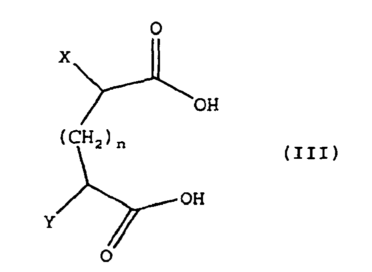 où X et Y sont tels que définis dans la revendication 1, dans un mélange contenant un solvant comme le dichlorométhane anhydre, la triéthylamine, II, III le dicyclohexylcarbodiimide (DCC) et la diméthylaminopyridine (DMAP), on obtient un composé de formule (IV) 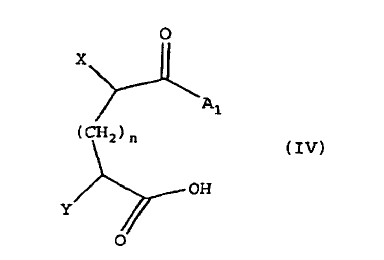 composé que l'on fait réagir dans les mêmes conditions que précédemment avec un composé de formule (V)
A₂ - H (V)
et on obtient le composé de formule (I).

## Claims

1. Bioprecursor of formula (I) 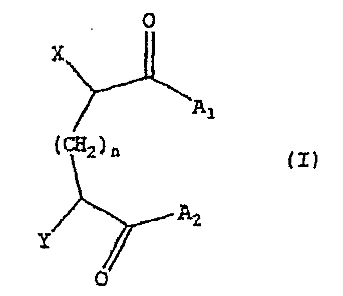 in which
A₁ represents a tocophenyl radical,
A₂ represents a radical derived from a molecule which is capable of being used in dermatology or cosmetology and is chosen from ceramides of plant origin, flavonoids, retinol, retinal, retinoic acid, vitamin D and calcitriol;
X and Y represent, independently of each other, a hydrogen atom, a hydroxyl group or a (C₁-C₂₀)alkyl group; and
n represents an integer between 0 and 10.

2. Bioprecursor according to Claim 1, **characterized in that** A₁ represents a tocopheryl radical and A₂ represents a radical chosen from the group comprising retinyl and cholecalciferyl radicals.

3. Bioprecursor according to either of Claims 1 and 2, **characterized in that** it is chosen from the group consisting of tocopheryl retinyl succinate and tocopheryl cholecalciferyl succinate.

4. Bioprecursor according to any one of Claims 1 to 3, **characterized in that** it is chosen from

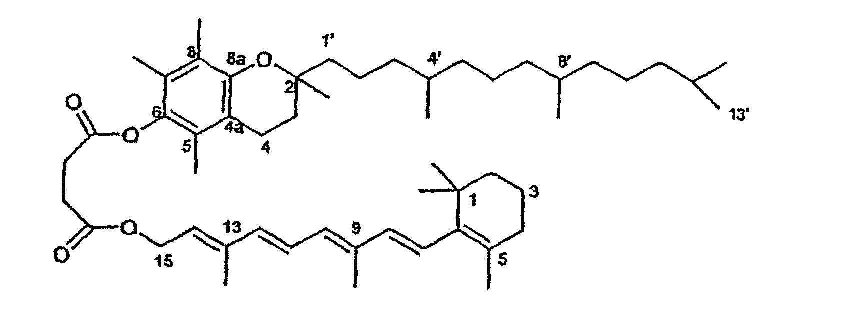

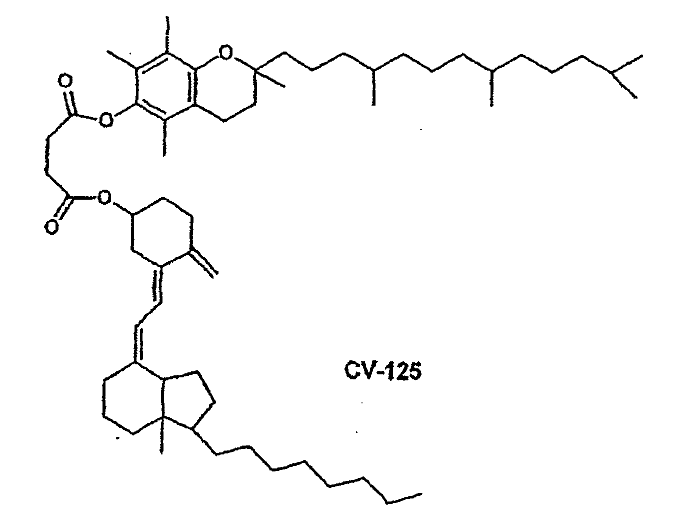

5. Pharmaceutical compositions, **characterized in that** they contain at least one bioprecursor according to any one of Claims 1 to 4 combined with a vehicle that is suitable for percutaneous administration.

6. Pharmaceutical compositions according to Claim 5, **characterized in that** they contain from 0.001% to 10% by weight and preferably 0.01% to 0.1% by weight of bioprecursors relative to the total weight of the composition.

7. Pharmaceutical compositions according to either of Claims 5 and 6, **characterized in that** they are in the form of an oil-in-water (O/W) or water-in-oil (W/O) emulsion.

8. Pharmaceutical compositions according to either of Claims 5 and 6, **characterized in that** they are in the form of spherules.

9. Pharmaceutical compositions according to Claim 7, **characterized in that** the proportion of the fatty phase ranges from 5% to 80% by weight and preferably from 5% to 50% by weight relative to the total weight of the composition.

10. Pharmaceutical compositions according to any one of Claims 6 to 9, **characterized in that** they also contain acceptable cosmetic or dermatological additives.

11. Process for preparing the bioprecursors according to any one of Claims 1 to 3, **characterized in that** a compound of formula (II)
A₁ - H (II)
in which A₁ is as defined in Claim 1,
is reacted with a compound of formula (III) 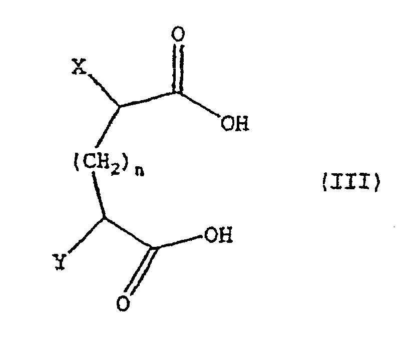 in which X and Y are as defined in Claim 1,
in a mixture containing a solvent, for instance anhydrous dichloromethane, triethylamine, II, III, dicyclohexylcarbodiimide (DCC) and dimethylaminopyridine (DMAP), to give a compound of formula (IV) 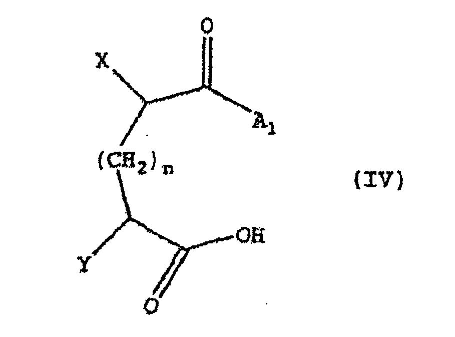 which compound is reacted under the same conditions as above with a compound of formula (V)
A₂ - H (V)
to give the compound of formula (I).

## Patentansprüche

1. Biovorstufe der Formel (I) 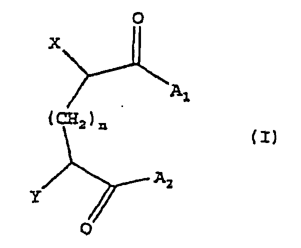 in der
A₁ einen Tocopherylrest darstellt,
A₂ einen Rest darstellt, der aus einem Molekül hervorgeht, das in der Dermatologie oder Kosmetik verwendet werden kann und ausgewählt ist aus Ceramiden pflanzlichen Ursprungs, Flavonoiden, Retinol, Retinal, Retinoesäure, Vitamin D, Calcitriol,
X und Y unabhängig voneinander ein Wasserstoffatom, eine Hydroxygruppe oder eine (C₁-C₂₀)-Alkylgruppe darstellen; und
n eine ganze Zahl zwischen 0 und 10 einschließlich darstellt.

2. Biovorstufe nach Anspruch 1, **dadurch gekennzeichnet, dass** A₁ einen Tocopherylrest darstellt und A₂ einen Rest darstellt, der ausgewählt ist aus der Gruppe umfassend den Retinyl-, Cholecalciferyl-Rest.

3. Biovorstufe nach irgendeinem der Ansprüche 1 bis 2, **dadurch gekennzeichnet, dass** sie ausgewählt ist aus der Gruppe bestehend aus Tocopherylretinylsuccinat und Tocopherylcholecalciferylsuccinat.

4. Biovorstufe nach irgendeinem der Ansprüche 1 bis 3, **dadurch gekennzeichnet, dass** sie ausgewählt ist aus

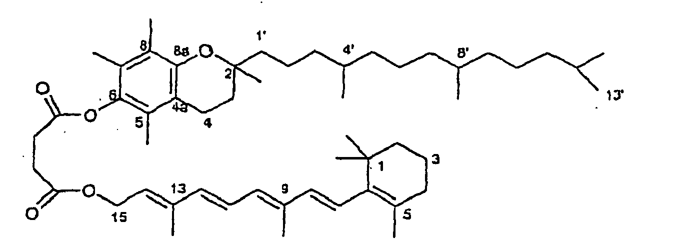

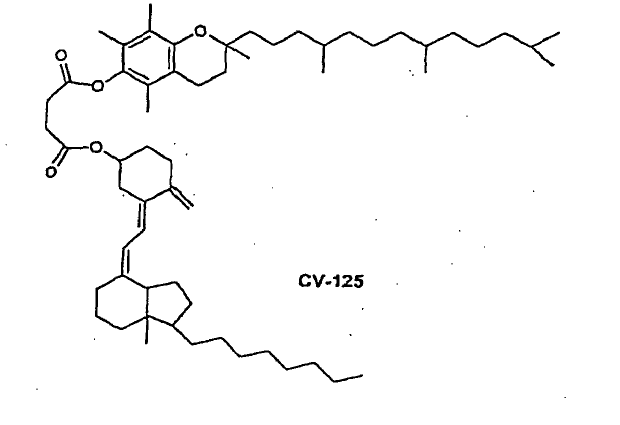

5. Pharmazeutische Zusammensetzungen, **dadurch gekennzeichnet, dass** sie mindestens eine Biovorstufe nach irgendeinem der Ansprüche 1 bis 4 in Verbindung mit einem für die perkutane Verabreichung geeigneten Vehikel enthalten.

6. Pharmazeutische Zusammensetzungen nach Anspruch 5, **dadurch gekennzeichnet, dass** sie 0,001 bis 10 Gew.-%, vorzugsweise 0,01 bis 0,1 Gew.-% Biovorstufen, bezogen auf das Gesamtgewicht der Zusammensetzung, enthalten.

7. Pharmazeutische Zusammensetzungen nach irgendeinem der Ansprüche 5 und 6, **dadurch gekennzeichnet, dass** sie in Form einer ÖI-in-Wasser (Ö/W)- oder Wasser-in-ÖI (W/Ö)-Emulsion vorliegen.

8. Pharmazeutische Zusammensetzungen nach irgendeinem der Ansprüche 5 und 6, **dadurch gekennzeichnet, dass** sie in Form von Kügelchen vorliegen.

9. Pharmazeutische Zusammensetzungen nach Anspruch 7, **dadurch gekennzeichnet, dass** der Anteil der Fettphase 5 bis 80 Gew.-%, vorzugsweise 5 bis 50 Gew.-% beträgt, bezogen auf das Gesamtgewicht der Zusammensetzung.

10. Pharmazeutische Zusammensetzungen nach irgendeinem der Ansprüche 6 bis 9, **dadurch gekennzeichnet, dass** sie darüber hinaus kosmetisch oder dermatologisch annehmbare Zusätze enthalten.

11. Verfahren zur Herstellung von Biovorstufen nach irgendeinem der Ansprüche 1 bis 3, **dadurch gekennzeichnet, dass** man eine Verbindung der Formel (II)
A₁ - H (II)
in der A₁ wie in Anspruch 1 definiert ist,
mit einer Verbindung der Formel (III) 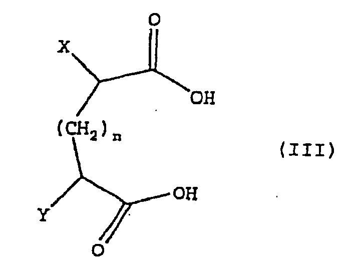 in der X und Y wie in Anspruch 1 definiert sind,
in einer Mischung, die ein Lösungsmittel wie wasserfreies Dichlormethan, Triethylamin, II, III, Dicyclohexylcarbodiimid (DCC) und Dimethylaminopyridin (DMAP) enthält, reagieren lässt,
man eine Verbindung der Formel (IV) 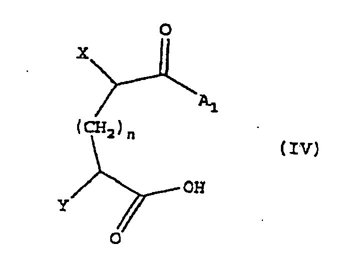 erhält, welche man unter den gleichen Bedingungen wie vorstehend mit einer Verbindung der Formel (V)
A₂ - H (V)
reagieren lässt, und man die Verbindung der Formel (I) erhält.
